# EUROPEAN PATENT SPECIFICATION

(11) **EP 1 911 404 B1**
(45) Date of publication and mention of the grant of the patent: **13.05.2009**
(21) Application number: 06291612.7
(22) Date of filing: 13.10.2006
(51) Int. Cl.: A61B 17/064, A61B 17/068

(54) **Instrument for storing and dispensing a surgical fastener**
Vorrichtung zur Aufbewahrung und Platzierung von chirurgischen Aufsätzen
Appareil de stockage et de pose d'attaches chirurgicales

(43) Date of publication of application: 16.04.2008
(73) Proprietor: SOFRADIM PRODUCTION, 01600 Trévoux (FR)
(72) Inventor: Bailly, Pierre, 69300 Calluire (FR); Perdreaux, David, 01140 Saint Etienne dur Chalaronne (FR); Therin, Michel, 69004 Lyon (FR)
(74) Representative: Brédeville, Odile Marie

(56) References cited:
- WO-A-03/075773
- FR-A- 2 876 020
- US-A1- 2005 187 565

## Description

The present disclosure relates to a device for deploying and ejecting a recumbent I-shaped surgical fastener, the fastener comprising an anchoring bar, a stop bar and a linking strip. The device is configured for attachment at a distal end of an instrument for storing, dispensing and positioning such fasteners.

WO 03/075773 already discloses an instrument for dispensing and positioning surgical fasteners with the aim of fastening the fabric of a prosthesis to human tissue, for example the abdominal wall of a patient. In that document, the fasteners are positioned by means of a hollow, bevelled needle, which passes through the prosthesis and flesh.

Also disclosed, in document FR 2 876 020, is another instrument for dispensing and positioning surgical fasteners, which corresponds to the preamble of claim 1, in which the needle is replaced with an ejection guide, the distal end of which is straight and not bevelled. Penetration of a fastener into flesh is ensured by the shape of the distal part of the anchoring bar, namely a conical shape terminating in a hemispherical shape.

At rest the ejection guide is housed in the tubular body of the instrument and the positioning of the fasteners is accompanied by the translational movement of the ejection guide towards the distal end of the instrument, the length of the projecting part of the ejection guide at the moment of ejection being equal to 3 mm or less. The fasteners, initially in the form of a recumbent I, are unfolded inside the ejection guide, therefore inside the tubular body of the instrument, and it is only once the stop bar is straightened up that it leaves the tubular body.

Such an instrument may be used by introducing the tubular body into a trocar. The tendency is to use trocars having the smallest diameter possible, for example around 5 mm. The diameter of the tubular body is therefore reduced accordingly. It follows that the fasteners, which must have dimensions sufficient to exert their retention function on the prosthesis in human tissue, are forced, when they are being stored in this tubular body, to be even more folded-up. Their deployment is therefore more tricky and, in addition, it cannot take place in the tubular body, the diameter of which is smaller than the size of an unfolded fastener.

Document US 2005/0187565 discloses a I-shaped fastener comprising an anchoring bar, stop bar and a linking strip.

One object of the present disclosure is to provide a device for deploying and ejecting a recumbent I-shaped surgical fastener, which allows satisfactory deployment of the fastener even when it is stored in a small-diameter tubular body.

For this purpose, and according to a first aspect, the disclosure relates to a device for deploying and ejecting a recumbent I-shaped surgical fastener. The fastener comprises an anchoring bar, a stop bar and a linking strip, the device being intended to be positioned to the distal end of an instrument for storing, dispensing and positioning such fasteners. The device has a longitudinal axis and a distal end that lies in a plane approximately orthogonal to the longitudinal axis and includes a first longitudinal channel emerging at the proximal and distal ends of the device, in which the anchoring bar of a fastener can slide longitudinally up to a point where it is ejected at the distal end of the device, the first channel having a longitudinal slot intended for passage of the linking strip of said fastener during said sliding.

According to a general definition of the disclosure, the device has an approximately longitudinal bearing face that defines an open half-space. The device further includes:
- a second longitudinal channel in which the stop bar can slide towards the distal end of the device, the second channel lying approximately in the same plane as the first channel and the slot of the first channel, emerging at the proximal end of the device and extending into the open half-space against the bearing face;
- a first cam that closes off the distal end of the second channel and designed to form a stop surface for the distal end of the stop bar and to allow sliding from said distal end in the opposite direction to the first channel so as to produce a pivoting movement of the stop bar;
- at least one approximately transverse splaying cam, located proximal to the first cam, capable of cooperating with the linking strip and/or the stop bar in order to cause a lateral splaying movement of the proximal part of the stop bar away from the bearing face; and
- an approximately transverse escapement cam, located between the first cam and the one or more splaying cams, capable of cooperating with the distal part of the stop bar in order to allow lateral displacement of the distal part of the stop bar away from the bearing face, and its disengagement from the first cam.

When the deployment of the ejection device is fixed to the distal end of the instrument, the open half-space lies outside the tubular body of this instrument. It is in this half-space that the deployment of the fastener takes place, which is therefore not impeded by the tubular body of restricted dimensions.

This arrangement, in combination with the fact that the first cam is shaped so as to allow the distal end of the stop bar to slide away from the first channel, allows the stop bar to pass beyond the diameter of the deployment/ejection device, both downwards (away from the first channel) and upwards. This was not possible with the instruments of the prior art, in which there was only a means of blocking the distal end of the stop bar, which did not allow the latter to slide downwards - it was therefore necessary for the tubular body to have a large diameter, especially so as to permit the stop bar to pivot without the proximal end of the latter being blocked in this movement by butting against the internal face of the tubular body.

Moreover, owing to the fact that the distal end of the device lies in a plane approximately orthogonal to the longitudinal axis, the device is capable of dispensing the fasteners with minimum trauma.

The first cam may generally lie in a plane orthogonal to the bearing face and is inclined, on going away from the first channel, from the proximal end of the device towards the distal end.

Advantageously, the first cam has, seen in cross section in a longitudinal plane orthogonal to the bearing face, a concave shape. It thus forms a gutter that allows the distal end of the stop bar to be guided during its sliding movement, while preventing it from escaping.

In one embodiment, the device may comprise two splaying cams designed to cause two sequenced lateral splaying movements of the proximal part of the stop bar.

The device may also include a longitudinal rib projecting approximately at right angles from the bearing face. The proximal face of said rib forms a first ramp constituting a splaying cam. Furthermore, the rib may have a distal part of greater height than its proximal part, the proximal face of the distal part of the rib forming a second ramp which, located distal of the first ramp, constitutes the escapement cam.

In one embodiment, the device includes a cavity formed in the bearing face, in line with the first channel, proximal of the proximal face of the rib, the distal face of which constitutes a subsequent splaying cam.

Moreover, the first channel may have a proximal part of larger transverse dimension than the distal part, a side wall of the proximal part of the first channel forming a ramp over which the distal end of the anchoring bar of a fastener can slide in order to cause the lateral displacement of said anchoring bar towards the distal part of the first channel.

In embodiments, the device may have an approximately cylindrical shape, with a diameter less than 6 mm. It may further include a proximal cylindrical appendage of smaller diameter, intended to be inserted into the distal end part of an instrument for storing, dispensing and positioning surgical fasteners.

In one embodiment, the distal end of the device includes a transverse wall substantially flanking the distal outlet of the first channel. The material added by the presence of this transverse wall provides further protection of the device and minimizes the thrust of the distal end of the device against human tissue when the fastener is being positioned.

According to a second aspect, the disclosure relates to an instrument for storing, dispensing and positioning recumbent I-shaped surgical fasteners. The fasteners include an anchoring bar, a stop bar and a linking strip. The instrument includes a gripping means provided with an actuator that can move on a body between a pushed-out position and a pushed-in position. The instrument further includes an elongate tubular body in which fasteners are stored longitudinally one behind another. The tubular body is fixed to the body of the gripping means and further includes a device for deploying and ejecting a fastener as described above. The device is fixed to the distal end of the tubular body so that the open half-space is located outside the tubular body.

In one embodiment the instrument includes:
- a stationary component housed in the tubular body and a moving component housed in the tubular body so that it can be moved in longitudinal translation with respect to the stationary component by means of an advancing rod that can be actuated by the actuator. The stationary and moving components are positioned along a longitudinal face and each having notches provided in said longitudinal face, with a pitch corresponding to the length of the stop bar of a fastener; and
- a slide placed between the longitudinal faces of the stationary and moving components, having an approximately planar main part, a distal part that bears against the proximal end of the stop bar of the proximalmost fastener and, in its proximal part, a first resilient tongue projecting towards the moving component and a second resilient tongue projecting towards the stationary component, each capable of being engaged in a corresponding notch. The geometry of the notches is designed so that the displacement of the moving component with respect to the stationary component towards the distal end of the instrument causes, by means of the first tongue being engaged in a notch of the moving component, the displacement of the slide in the same direction, until the second tongue of the slide is engaged in a notch of the stationary component. The cooperation between the second tongue and a notch of the stationary component prevents the slide from returning towards the proximal end of the instrument when the moving component is displaced relative to the stationary component towards the proximal end of the instrument.

The stationary and moving components may have the form of semicylinders, the moving component including a radial orifice into which the curved end of the advancing rod is inserted.

According to one embodiment, the instrument includes, fixedly housed in the distal part of the tubular body, a magazine for storing the fasteners. The magazine includes a first longitudinal housing that receives the aligned anchoring bars of the fasteners and, contiguously, an ejection rod that can be actuated by the actuator. The magazine further includes a second longitudinal housing that receives the aligned stop bars of the fasteners, and a third housing, which joins the first and second housings, is configured for passage of the linking strips.

The gripping means may include:
- first and second levers that can be moved, by pushing in the actuator, between a proximal rest position and a distal end position. Movement to the end position causes longitudinal displacement in the distal direction of a rod for ejecting the distalmost fastener out of the deployment/ejection device and of a rod for advancing the fasteners in the tubular body, respectively;
- a member mounted so as to pivot about a pin attached to the body of the gripping means, which member includes a tooth capable of cooperating with a rack formed on one of the levers during displacement of the actuator; and
- a flexible return means, one end of which is connected to the pivoting member and the other end of which is connected to the body of the gripping means. The elastic return means and the profile of the tooth and the rack are designed so that:

- when the actuator is pushed in as far as an intermediate pushed-in position, the elastic return means urges the pivoting member towards a position such that the cooperation between the tooth of the pivoting member and the rack of the lever prevents the actuator from returning to the pushed-out position from said intermediate pushed-in position; and
- after the levers have reached their distalmost end position, upon releasing the actuator as far as an intermediate release position, the flexible return means urges the pivoting member towards a position such that the cooperation between the tooth of the pivoting member and the rack of the lever prevents the actuator from being pushed in from said intermediate release position.

A double non-return system may advantageously be obtained.

Advantageously, the rack of the lever includes, at its distal end, a tooth of larger size and the pivoting member includes, on one side, a finger and, on the other side, a tab that is capable of cooperating with a stop attached to the body of the gripping means when the finger comes into contact with the larger tooth, so that, when the actuator has been pushed in, an elastic deformation of the tab of the pivoting member in contact with the stop is necessary in order for the finger to pass beyond said larger tooth. When the actuator is pushed in, the user therefore receives a tactile indication that he has reached the fully pushed-in position of the actuator, and therefore that the fastener has been suitably ejected.

One embodiment of the disclosure will now be described with reference to the appended figures, in which:
- Figure 1 is a side view of an instrument for storing, dispensing and positioning surgical fasteners according to an embodiment of the invention;
- Figure 2 is a side view of a recumbent I-shaped surgical fastener in the storage position;
- Figures 3 and 4 are perspective views, from the rear and front respectively, of a device for deploying and ejecting a surgical fastener according to an embodiment of the invention;
- Figure 5 is a sectional view of the deployment/ejection device, in a longitudinal plane;
- Figures 6 to 15 illustrate successive steps in the deployment and ejection of a fastener;
- Figure 16 is a side view showing the mechanism of the instrument, housed inside the gripping means;
- Figure 17 is a perspective view of the slide in the tubular body for advancing the fasteners;
- Figure 18 is a partial schematic view of a series of fasteners that are stored in a magazine, and of the distal end of the slide of Figure 17;
- Figure 19 is a partial cross-sectional view of the tubular body in the region that receives the magazine;
- Figures 20 to 21 are schematic representations in a lateral sectional view of the fastener-advancing system;
- Figures 23 and 24 are detailed views of the inside of the gripping means, showing the non-return system of the actuator;
- Figures 25 to 27 are detailed views of the inside of the gripping means, showing the system for indicating the end of push-in travel of the actuator;
- Figure 28 is a perspective view of an alternative embodiment of the deployment/ejection device; and
- Figure 29 is a side view of another exemplary fastener which does not form part of the invention.

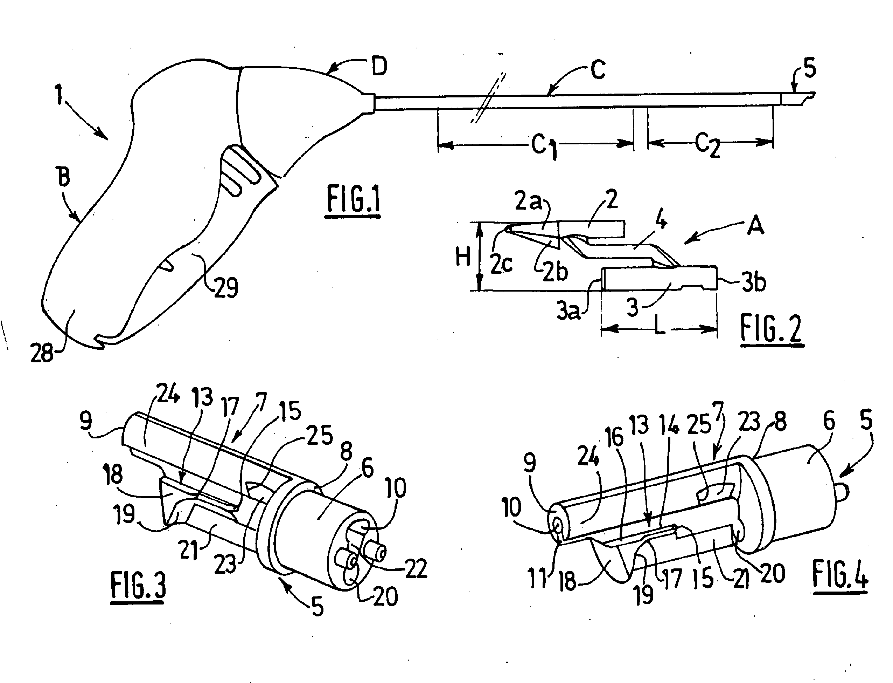

The instrument 1 shown in Figure 1 is designed to dispense fasteners A which have, as shown in Figure 2, the general shape of a recumbent "I" and are composed of two parallel bars, namely an anchoring bar 2 and a stop bar 3, joined together by a linking strip 4. The fastener A is made of plastic, especially a biocompatible and preferably bioresorbable material.

The distal part 2a of the anchoring bar 2 has a conical shape. This distal part 2a may include a notch 2b projecting towards the stop bar 3 in order to protect the anchoring bar 2/linking strip 4 intersection, which is stressed at the moment when the fastener A is ejected and positioned. Furthermore, the distal end 2c of the anchoring bar 2 has a hemispherical shape.

The term "proximal" relates to a location closer to the user of the instrument 1, while the term "distal" relates to a location further from the user.

The stop bar 3 has a distal end 3a and a proximal end 3b, and has a length L.

The linking strip 4 is joined to the anchoring bar 2, approximately at the center of the latter, and is joined to the stop bar 3 in a region close to the center of the stop bar 3, but slightly offset towards its proximal end 3b. In one embodiment, the length L of the anchoring bar may measure 6.5 mm and the anchoring bar/linking strip join is offset by about 0.5 mm with respect to the center of the anchoring bar, towards the distal end of the anchoring bar.

The proximal part, or alternatively the distal part, of the stop bar 3 is defined as that part of the stop bar 3 which is located between its proximal end 3b, or alternatively its distal end 3a, and the linking region between the stop bar 3 and the linking strip 4.

In the storage position in the instrument 1, the fastener A is folded so that the linking strip 4 has two curved regions (in the vicinity of its points of attachment to the anchoring bar 2 and the stop bar 3), and a central zone approximately parallel to the bars 2, 3. In this position, illustrated in Figure 2, the height H of the fastener may be about 5 mm or less. In embodiments, the height H of the fastener is no more than 4 mm.

As shown in Figure 1, the instrument 1 includes a gripping means B to which a tubular body C having a longitudinal axis is fixed. A socket D surrounds the junction region between these two elements. From its proximal end, the tubular body C includes, as will be seen later, a first zone C1 containing a step-by-step system for advancing the fasteners A followed by a second zone C2 in which the fasteners A are stored.

Fixedly mounted on the distal end of the tubular body C is a device 5 for deploying and ejecting the fasteners A, said device projecting beyond the distal end of the tubular body C.

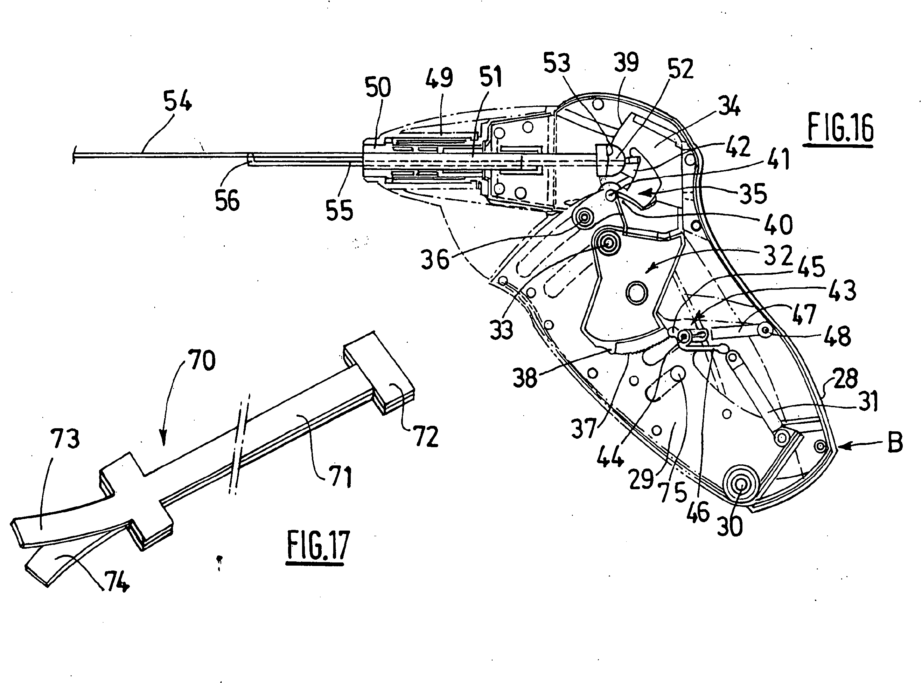

The longitudinal mid-plane of the instrument 1 is defined as the plane passing through the axis of the tubular body C and dividing the gripping means B into two approximately identical parts (corresponding to the plane in which the gripping means B is shown in Figure 16). The term "transverse" denotes a direction generally perpendicular to this plane, or a plane orthogonal to the longitudinal axis of the tubular body C.

The deployment/ejection device 5 will now be described with reference to Figures 3 to 5.

The device 5 has an approximately cylindrical shape, with a diameter of about 5 mm or less. In the example shown, it is formed from a single metal component. In one embodiment the device may be made of plastic and/or formed from several components.

The proximal part of the device 5 forms a cylindrical appendage 6 intended to be introduced into the tubular body C. The distal part 7 of the device 5 has a larger diameter, approximately identical to the outside diameter of the tubular body C, and thus defines a shoulder 8 that butts against the distal end of this body C.

The distal part 7 has the form of a semicylinder bounded by a bearing face 76 which, in the position in which the device 5 is fitted onto the instrument 1, lies generally in the longitudinal mid-plane of the instrument. Thus defined is an open half-space - except upstream - which allows the fastener A to be satisfactorily deployed. The distal end 9 of the device 5 is approximately planar and orthogonal to the longitudinal axis. This distal end 9 of the device 5 may be slightly domed, provided that its mean plane is generally orthogonal to the longitudinal axis of the device 5.

The device 5 includes a first longitudinal channel 10 offset with respect to the longitudinal axis of the device 5. For the sake of simplifying the description, it will be considered hereafter that the first channel 10 is located in the top of the device 5, recognizing that this device 5, once fitted onto the instrument 1, may adopt various positions in space during use.

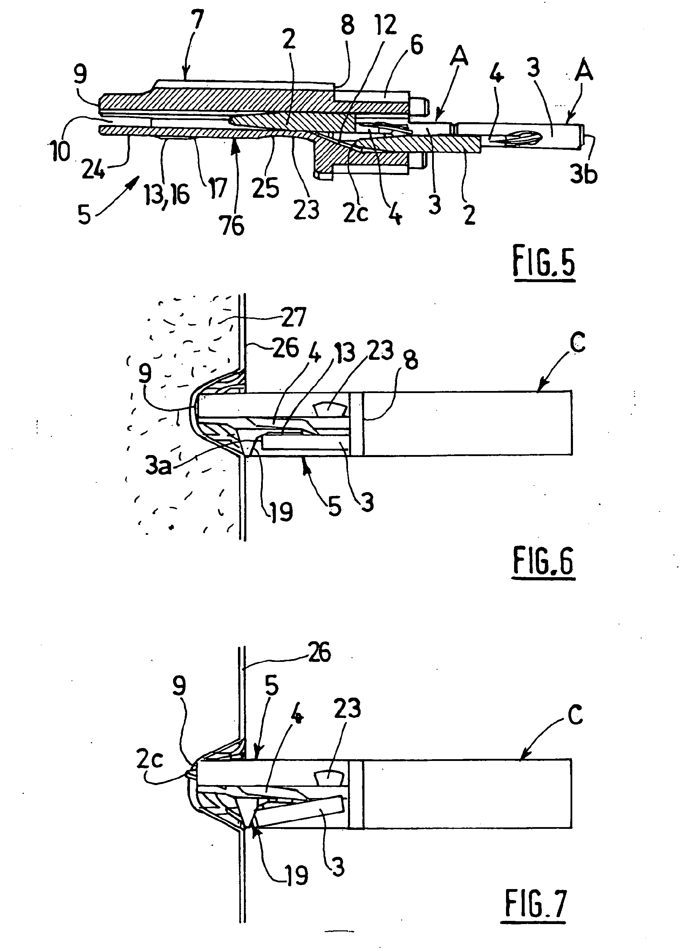

In the proximal part of the device 5, the first channel 10 has a larger cross section, for reasons that will be explained later. As shown in Figure 5, which is a sectional view in a plane passing through the longitudinal axis of the device and orthogonal to the longitudinal mid-plane, the junction zone between the proximal and distal parts of the first channel 10 forms a ramp 12 inclined from the proximal end towards the distal end, and towards the axis of the cylindrical distal part of the first channel 10.

The lower zone of the distal part 7 of the device 5 has a shorter length than the first channel 10. This lower zone includes a longitudinal rib 13, lying below the slot 11, substantially along the longitudinal axis of the device 5 and extending substantially from the middle of the distal part 7 right to the distal end of the lower zone. The rib 13 includes a proximal part 14, the proximal face 15 of which forms a ramp, a higher distal part 16 of the rib 13 has proximal face 17 which also forms a ramp.

A protuberance 18 on the distal part 16 of the rib 13 extends downwards and has a triangular shape in side view. A proximal face 19 of protuberance 18 is orthogonal to the longitudinal mid-plane and inclined downwards from the proximal end of the device 5 towards the distal end 9. In addition, this proximal face 19 has a concave shape, seen in section in a longitudinal plane orthogonal to the longitudinal mid-plane.

The device 5 further includes a second longitudinal channel 20, offset downwards with respect to the longitudinal axis of the device 5. In the distal part of the device 5, the second channel 20 is opened laterally and defined by a zone 21 set back with respect to the longitudinal mid-plane lying beneath the rib 13. In the proximal part of the device 5, the second channel 20 is approximately cylindrical and joined to the first channel 10 by a longitudinal housing 22 of downwardly elongate cross section.

The device 5 includes a cavity 23 provided in the wall 24 of the first channel 10, in the distal part 7 of the device 5, upstream of the proximal face 15 of the rib 13 and close to the shoulder 8. The cavity 23 has a distal face 25 generally orthogonal to the longitudinal mid-plane.

The description now refers to Figures 6 to 15 which show the various phases resulting in the deployment and ejection of a fastener A out of the instrument 1 by means of the device 5, with a view to fixing a prosthesis 26 to human tissue 27.

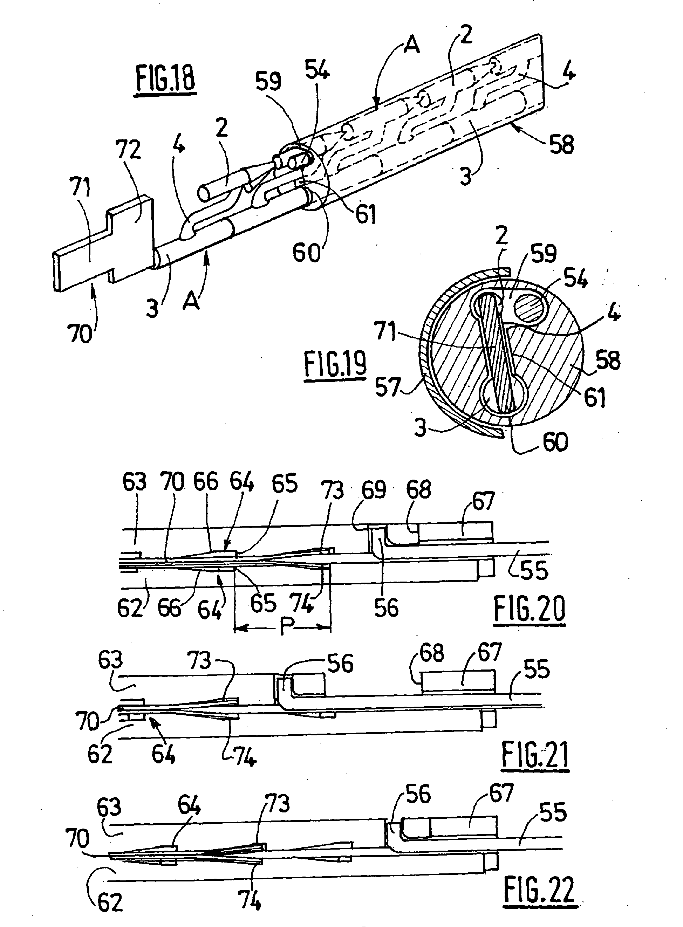

As will be seen later, Figures 5 to 15 the fasteners A are stored in the tubular body C one behind another with the anchoring bars 2 lying approximately along the axis of the first channel 10 and the stop bars 3 along the axis of the second channel 20 (see Figures 5 and 18).

As illustrated in Figure 5, while the fasteners A are being advanced towards the distal end 9 of the device 5, the distal end 2c of the anchoring bar 2 of the fastener A furthest downstream butts against the ramp 12 which slides over the latter, thus causing the lateral displacement of said anchoring bar 2 towards the distal part of the first channel 10. The anchoring bar 2 is then pushed towards the distal end 9 of the device 5, in a longitudinal sliding movement in the first channel 10, during which the linking strip 4 slides in the slot 11 and the stop bar 3 is made to slide in the second channel 20.

When the distal end 3a of the stop bar butts against the proximal face 19 of the protuberance 18, which forms a first cam surface (Figure 6), this distal end 3a slides downwards against the proximal face 19, being guided by the shape of the latter. This pivots the stop bar (Figure 7) which begins to straighten, while the anchoring bar 2 starts to project from the device 5.

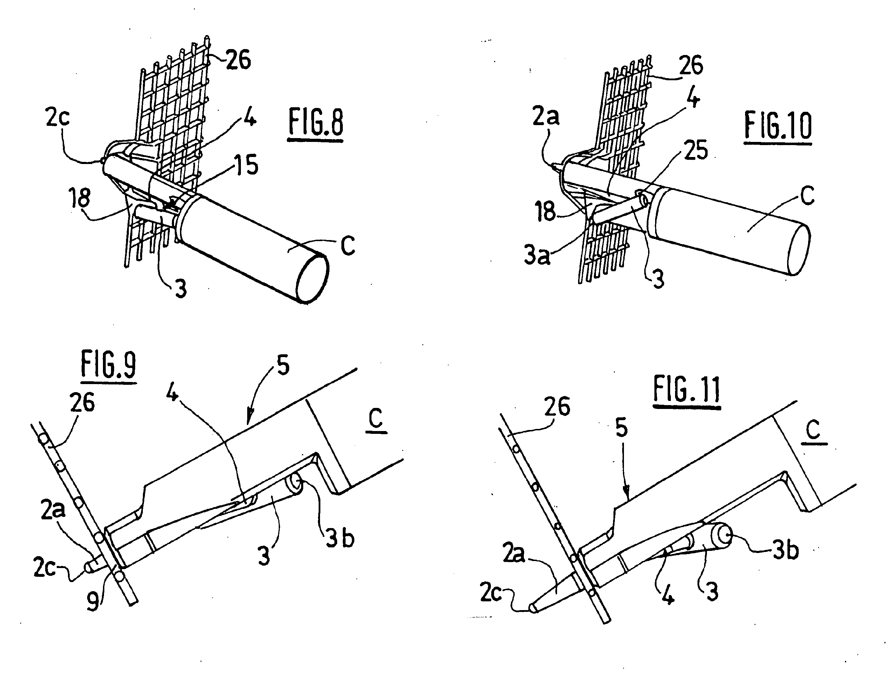

The junction between the linking strip 4 and the stop bar 3 is then in contact with the proximal face 15 of the rib 13, forming a second cam 15 (Figure 8). Second cam 15 causes a first lateral splaying movement of the proximal part of the stop bar 3, as shown in top view in Figure 9.

The proximal part of the stop bar 3 then comes into contact with the cavity 23 (Figure 10). As the sliding of the anchoring bar 2 continues, the distal part of the stop bar 3 continues to slide downwards against the proximal face 19. The angle of pivoting of the stop bar 3 progressively increases and the proximal part of the stop bar 3 cooperates with the distal face 25 of the cavity 23, forming a third cam 25. As a result, the lateral splaying of the proximal part of the stop bar 3 increases (Figure 11). This lateral splaying allows the stop bar 3 to avoid the lower face of the first channel 10, which would otherwise prevent the continuation of the pivoting movement.

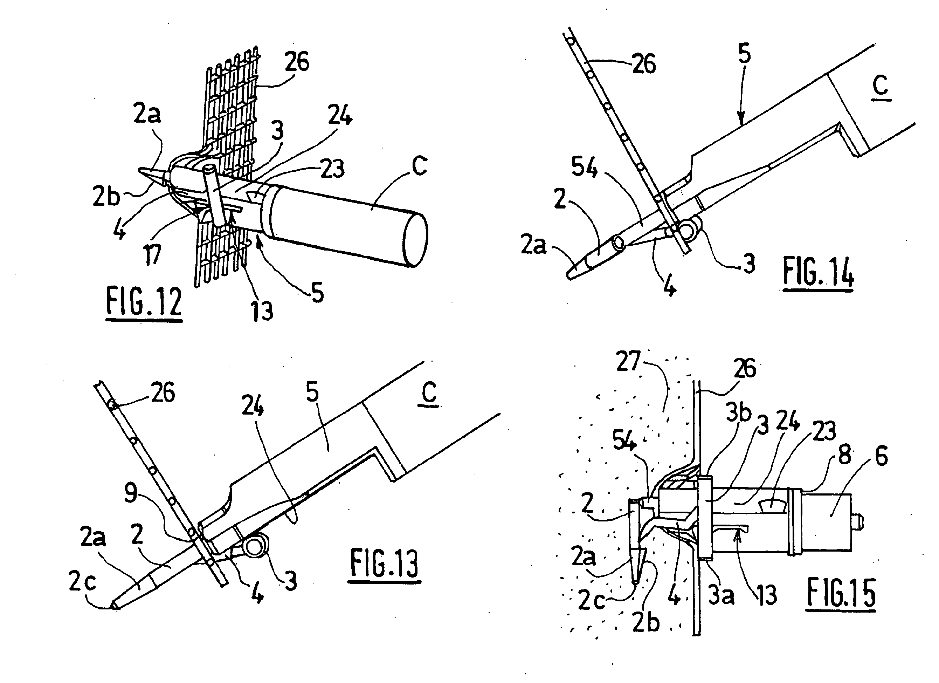

Next, the proximal part of the stop bar 3 slides against the wall 24 of the first channel 10 until the distal part of the stop bar 3 comes into contact with the proximal face 17 of the distal part 16 of the rib 13, forming a fourth cam 17 (Figure 12). This movement results in the lateral displacement of the distal part of the stop bar 3, which escapes from the proximal face 19. The stop bar 3 is then approximately upright (Figure 13). The continuation of the thrusting movement of the anchoring bar 2 results in it being completely ejected from the device 5 (Figure 14) and then pivoted, at least through an angle of at least 30° with the linking strip 4 and preferably an angle ranging up to 90° with this linking strip 4. Preferably, the anchoring bar 2 pivots until it is approximately parallel to the upright stop bar 3, which is pressed against the prosthesis 26, the linking strip 4 then being approximately perpendicular to the two bars 2, 3 and the fastener A adopting an upright "I" configuration. In this position (Figure 15), the fastener A holds the prosthesis 26 against the human tissue 27.

The instrument 1 will now be described in greater detail.

As illustrated in Figure 16, the gripping means B includes a body 28, made in two symmetrical parts assembled by force-fitting them or by means of rivets or screws, and an actuator 29 mounted so as to move on the body 28 in an articulated fashion about a transverse pin 30 of the body 28, between a pushed-in position and a pushed-out position. A spring 31 urges the actuator 29 towards its pushed-out position.

Housed in the body 29 are the following:
- a first control lever 32 articulated to a transverse pin 33 of the body 28, the upper part of said control lever 32 including two projections 34 separated laterally from each other (only one of them being visible in Figure 16); and
- a second control lever 35 articulated to a transverse pin 36, housed between the two projections 34 of the first lever 32.

The first lever 32 has a circularly arcuate lower end provided with rack 37 and, in its distal part, with a larger tooth 38. These projections 34 have a cam-shaped distal face 39. Furthermore, the first lever 32 includes, in the central part, a projection 40 capable of cooperating with a transverse lug 41 provided on the second lever 35. The latter includes a cavity, the bottom of which forms a transverse cam 42.

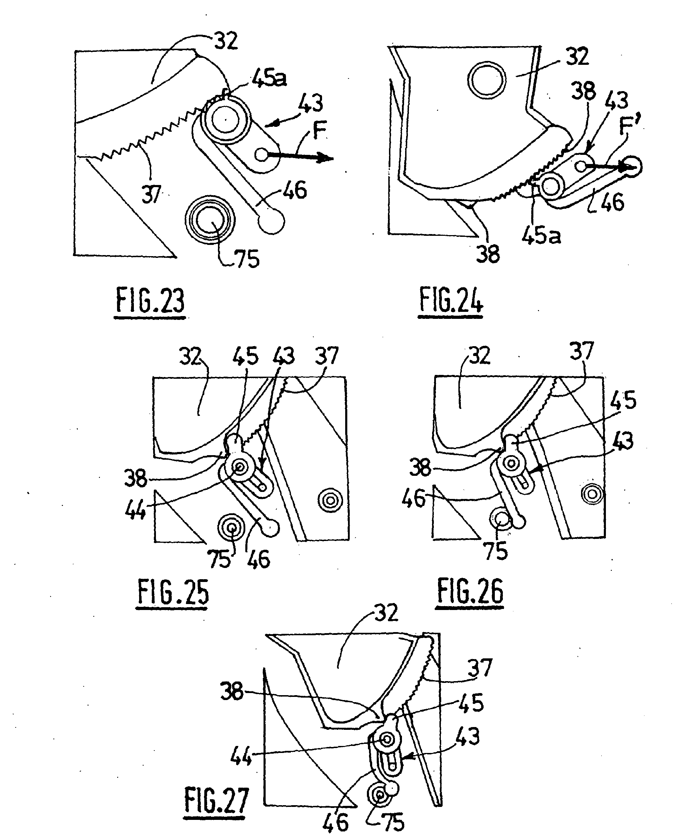

The body 28 of the gripping means B also contains a pivoting member 43 articulated to a transverse pin 44 of the body 28. This pivoting member 43 includes a tooth 45a (visible in Figures 23 and 24) capable of cooperating with the rack 37 of the first lever 32, a finger 45, larger than the tooth 45a, and a tab 46 projecting away from the finger 45. A spring 47 links the pivoting member 43 to a transverse pin 48 of the body 28.

The gripping means B is completed, inside the socket D, with a cylindrical ferrule 49 in which, inside a spacer 50, a piston 51 is fitted. The piston 51 has a thinner proximal end part 52, introduced between the two projections 34 of the first lever 32 and, on either side of the proximal end part 52 and distal thereof, two curve surfaces 53 that can cooperate with the cam 39 of the projections 34.

An ejection rod 54 is fixed (for example welded) to the distal end of the piston 51, near its periphery. This rod 54 extends longitudinally, approximately as far as the distal end of the tubular body C. Moreover, the piston 51 has an approximately central longitudinal bore in which an advancing rod 55 can slide. The advancing rod 55 has a proximal end that can cooperate with the cam 42 of the second lever 35 and a curved distal end 56, located in the zone C1 of the tubular body C.

The tubular body C includes a hollow metal envelope 57 (cf. Figure 19) in which various elements are housed.

Fixedly mounted in the second zone C2 of the tubular body C is a magazine 58 for storing the fasteners A. As illustrated in Figures 18 and 19, this magazine 58 comprises a first longitudinal housing 59 that receives the aligned anchoring bars 2 of the fasteners A and, contiguously, the ejection rod 54, a second longitudinal housing 60 that receives the aligned stop bars 3 of the fasteners A, and a third housing 61, which joins the first and second housings 59, 60, for passage of the linking strips 4. These housings 59, 60 and 61 are placed in alignment with the channels 10, 20 and with the housing 22 (cf. Figure 3) that are provided in the device 5. It should be noted that, in Figure 18, the ejection rod 54 and the magazine 58 have been truncated in their proximal part in order to make it easier to understand the figure, all the fasteners A being in fact housed in the magazine 58. The magazine 58 may be made in several sections joined together longitudinally.

The first zone C1 of the tubular body C contains a step-by-step advancing system for the fasteners A, which system will now be described with reference to Figures 17 to 22.

Component 62 is fixed in the envelope 57 of the tubular body C and is stationary relative to the tubular body C. Component 63, which can move relative to the stationary component 62 is also positioned in the envelope 57. Both these components have the shape of semicylinders superposed along their longitudinal face (Figure 20).

Provided in the longitudinal face of each of the components 62, 63 are notches 64, arranged with a pitch P approximately equal to the length L of the stop bar 3 of a fastener A. Each notch 64 has an upright transverse proximal edge 65 and a distal edge 66 inclined to the longitudinal axis of the tubular body C towards the periphery of the latter, upon moving away from the distal end of the tubular body C towards this proximal end.

The stationary component 62 has a proximal cylindrical part 67 that defines a transverse stop surface 68. The moving component 63 includes, near its proximal end, a radial orifice 69 into which the curved end 56 of the advancing rod 55 is inserted. In addition, a channel (not shown) is provided in the moving component 63 and in the proximal cylindrical part 67 of the stationary component 62, in order to house the ejection rod 54.

Placed between the longitudinal faces of the stationary component 62 and the moving component 63 is a slide 70, shown in Figure 17. The slide 70 comprises an approximately planar main part 71, a wider distal part 72 and, in its proximal part, first and second splayed-apart resilient tongues 73, 74. The slide 70 is for example formed from a metal sheet, cut and then folded onto itself, which may be bonded at the main 71 and distal 72 parts, but not at the tongue 73, 74, which are curved away from each other.

Initially, before the first use of the instrument 1, the slide 70 is mounted such that the first tongue 73, projecting towards the moving component 63, is engaged in the proximal notch 64 of component 63, and the second tongue 74, projecting towards the stationary component 62, is engaged in the proximal notch 64 of component 62. The moving component 63 is in contact with the transverse stop surface 68 of the stationary component 62, and the notches 64 of the components 62, 63 face each other in pairs. The distal part 72 of the slide 70 is engaged in the third housing 61 (Figure 19), bearing against the proximal end 3b of the stop bar 3 of the proximalmost fastener A.

The operation of the instrument 1 will now be described.

When a user presses on the actuator 29, it causes the first lever 32 to start to pivot, which, through the cooperation between the projection 40 and the lug 41, results in the second lever 35 pivoting. Through the cooperation between the proximal end of the advancing rod 55 and the cam 42 of the second lever 35, the advancing rod 55 is displaced longitudinally downwards, taking with it the moving component 63, which slides longitudinally relative to the stationary component 62 (Figure 21). The mechanism is designed so that this displacement corresponds to the pitch P, and therefore to the length L of the stop bar 3 of a fastener A. This value is, for example, about 7 mm.

During this movement, the proximal edge 65 of the proximal notch 64 of the moving component 63 pushes the first tongue 73 downstream. The slide 70 is therefore displaced downstream (the second tongue 74 deforming elastically in order to come into the plane of the main part 71 of the slide 70) and, at the end of the movement, the second tongue 74 is housed in the notch 64 located immediately distal of the proximal notch of the stationary component 62.

The distal part 72 of the slide 70 has therefore pushed the train of fasteners A via the stop bars 3, distally, by the distance P, and therefore the distance L. Consequently, the distalmost fastener A has been displaced in the deployment and ejection device 5, and its anchoring bar 2 has slid over the ramp 12 so as to come into alignment with the ejection rod 54 (Figure 5).

When the user continues to push in the actuator 29, the second lever 35 remains immobile, as therefore do the advancing rod 55, the moving component 63, the slide 70 and the fasteners A (with the exception of the most downstream one). However, the pivoting of the first lever 32 continues, which, through the cooperation between the cams 39 of the projections 34 and the curve surfaces 53 of the piston 51, results in the ejection rod 54 sliding longitudinally downstream. It should be noted that the sliding of the rods 54, 55 is not the same, this being made possible by the fact that the advancing rod 55 slides in the piston 51 to which the ejection rod 54 is fixed.

The distal end of the ejection rod 54 then pushes the anchoring bar 2 of the fastener A located furthest downstream into the deployment/ejection device 5. Via the steps described above (Figures 6 to 15), the anchoring bar 2 is ejected into the tissue 27, while the stop bar 3 pivots and the fastener A is deployed. This deployment takes place in the open half-space of the device 5, and therefore outside the tubular body C. The longitudinal travel of the ejection rod 54 is, for example, about 24 mm.

The user can then release the actuator 29. Various elastic return means (not shown) allow the rods 54, 55 to return to their initial positions, awaiting the next time the actuator 29 is pushed in.

When the advancing rod 55 slides proximally, it brings the moving component 63 back against the transverse stop surface 68 of the stationary component 62. However the second tongue 74, in abutment against the proximal edge 65 of the notch 64 of the stationary component 62, prevents the slide 70 from returning further proximal. In Figure 22, the slide 70 has therefore advanced by one pitch P distally relative to Figure 20, and it will therefore be able to advance the train of fasteners A distally by one pitch the next time the actuator 29 is pushed in.

Finally, the double non-return system with which the gripping means B is provided will be described with reference to Figures 16 and 23 to 27.

Before use, the pivoting member 43 is in the neutral position, as shown in Figure 16.

When the actuator 29 is pushed in as far as an intermediate position, wherein the tooth 45a of the pivoting member 43 cooperates with the rack 37 of the first lever 32, the spring 47 urges the pivoting member 43 (indicated by the arrow F in Figure 23) in such a way that the tooth 45a, engaged in the rack 37, prevents the actuator 29 from returning to the pushed-out position from said intermediate position. Thus, the user is forced to continue to press on the actuator in order for the procedure of advancing, deploying and ejecting the fastener A to be completely finished. It is important that the movement of pushing in the actuator be accomplished right to the end by the user so as to avoid damaging both the prosthesis and the instrument.

Towards the end of the movement of pushing in the actuator 29, the finger 45 of the pivoting member 43 comes into contact with the larger tooth 38 (Figure 25). This results in the pivoting member 43 pivoting about its pin 44 until the tab 46 comes into contact with a stop 75 integral with the body 28 of the gripping means B (Figure 26). An elastic deformation of the tab 46 of the pivoting member 43 in contact with the stop 75 is required for the finger 45 to pass beyond the larger tooth 38 after the actuator has been pushed in (Figure 27). This constitutes a hard point felt by the user, who can therefore know whether or not the end of travel has been reached and, consequently, whether the fastener A has been correctly ejected. In addition, when the finger 45 passes beyond the larger tooth 38, a sudden pivoting of the first lever 32 takes place, and therefore a sudden advance of the ejection rod 54. This impact is transmitted to the distalmost fastener A which makes it easier to eject and pivot it.

At this step, the pivoting member 43 returns to a neutral position, similar to that shown in Figure 16, but this time beyond the toothing 37.

Next, when the actuator 29 is released as far as an intermediate position, the spring 47 urges the pivoting member 43 (indicated by the arrow F' in Figure 24) in such a way that the tooth 45a, engaged in the rack 37, prevents the actuator 29 from being pushed in from said intermediate position. Thus, the user cannot start a new cycle (advance of the train of fasteners and ejection of the distalmost fastener) until the mechanism has been returned to its rest position. This prevents the mechanism from becoming blocked and guarantees successful ejection of each of the fasteners A.

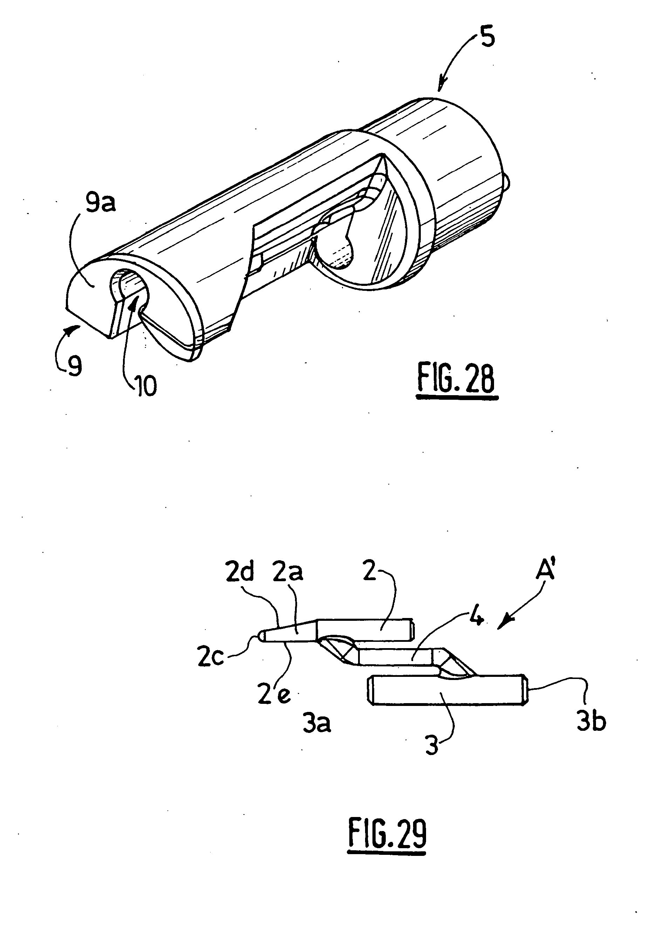

Another exemplary the fastener A', which does not form part of the invention, is shown in Figure 29. Fastener A' has no notch. Fastener A' of Figure 29 has the general shape of a recumbent "I" in the storage state. The references denoting the same elements as in Figure 2 have been preserved. The fastener A' comprises an anchoring bar 2 joined to a stop bar 3 via a linking strip 4. The fastener A' may be made of biocompatible and bioresorbable plastic.

The distal end 2c of the anchoring bar 2 has a hemispherical shape and the distal part 2a of the anchoring bar 2 has approximately the shape of a truncated cone, the external slope 2d of the cone, turned towards the outside of the fastener, being however more accentuated than the internal slope 2e, turned towards the stop bar 3. This accentuation of the external slope 2d of the cone allows the anchoring bar 2 of the fastener A' to pivot more naturally towards the stop bar 3 when the fastener is being positioned in tissue, as shown in Figures 13 to 15 - pivoting of the fastener A' is thus favored and its deployment towards an upright "I" configuration is thus facilitated.

In the distal part of the device 5, the first channel 10 is approximately cylindrical and has a longitudinal slot 11 opened downwards. This first channel 10 emerges at its distal end near the distal end 9 of the device 5. Figure 28 shows an alternative embodiment of the device 5 of Figure 4, in which the distal end 9 of the device 5 includes a transverse wall 9a substantially flanking the distal outlet of the first channel 10. The material added by the presence of this transverse wall 9a provides further protection of the device 5 and minimizes the thrust of the distal end 9 of the device 5 against human tissue when the fastener A is being positioned, for example during the steps shown in Figures 6 and 7.

The invention therefore provides a definite improvement over the prior art, by providing a device for deploying and ejecting surgical fasteners and an instrument for storing, dispensing and positioning them that can be used in small-diameter trocars, while still guaranteeing satisfactory deployment of the fasteners and, consequently, very good retention of a prosthesis against human flesh.

The invention is not limited to the embodiment described above by way of example, but on the contrary it encompasses all alternative embodiments thereof, as indicated by the appended claims.

## Claims

1. Device (5) for deploying and ejecting a recumbent I-shaped surgical fastener, the fastener having an anchoring bar (2), a stop bar (3) and a linking strip (4), the device (5) being intended to be positioned adjacent the distal end of an instrument (1) for storing, dispensing and positioning fasteners (A; A'), the device having a longitudinal axis and a distal end (9) that lies in a plane approximately orthogonal to the longitudinal axis and includes a first longitudinal channel (10) emerging at the proximal and distal ends of the device, in which the anchoring bar (2) of a fastener can slide longitudinally up to a point where it is ejected at the distal end of the device, the first channel (10) having a longitudinal slot (11) intended for passage of the king strip (4) of said fastener during said sliding the device further including a second longitudinal channel (20) in which the stop bar (3) can slide towards the distal end (9) of the device (5), the second channel (20) lying approximately in the same plane as the first channel (10) and the slot (11) of the first channel, emerging at the proximal end of the device and, a first cam (19) that closes off the distal end of the second channel (20), designed to form a stop surface for the distal end (3a) of the stop bar (3) and to allow sliding from said distal end (3a) in the opposite direction to the first channel (10), so as to produce a pivoting movement of the stop bar (3), **characterized in that** the device (5) has an approximately longitudinal bearing face (76) that defines an open half-space not containing the first channel (10) and wherein the second channel (20) extends into the open half-space against the bearing face (76);
and **in that** the device (5) further includes:
- at least one approximately transverse splaying cam (15, 25), located proximal to the first cam (19), capable of cooperating with the linking strip (4) and/or the stop bar (3) in order to cause a lateral splaying movement of the proximal part of the stop bar (3) away from the bearing face (76); and
- an approximately transverse escapement cam (17), located between the first cam (19) and the at least one splaying cam (15, 25), capable of cooperating with the distal part of the stop bar (3) in order to allow lateral displacement of the distal part of the stop bar (3) away from the bearing face (76), and its disengagement from the first cam.

2. Device (5) according to Claim 1, **characterized in that** the first cam (19) generally lies in a plane orthogonal to the bearing face (76) and is inclined, on going away from the first channel (10), from the proximal end of the device (5) towards the distal end (9) .

3. Device (5) according to Claim 1 or 2, **characterized in that** the first cam (19) has, seen in cross section in a longitudinal plane orthogonal to the bearing face (76), a concave shape.

4. Device (5) according to one of Claims 1 to 3, **characterized in that** it comprises two splaying cams (15, 25) designed to cause two sequenced lateral splaying movements of the proximal part of the stop bar (3) .

5. Device (5) according to one of Claims 1 to 4, **characterized in that** it includes a longitudinal rib (13) projecting approximately at right angles from the bearing face (76) and the proximal face (15) of said rib forms a first ramp constituting a splaying cam.

6. Device (5) according to Claim 5, **characterized in that** the rib (13) has a distal part (16) of greater height than its proximal part (14), the proximal face (17) of the distal part (16) of the rib (13) forming a second ramp which, located distal of the first ramp (15), constitutes the escapement cam (17).

7. Device (5) according to Claim 5 or 6, **characterized in that** it includes a cavity (23) formed in the bearing face (76), in line with the first channel (10), proximal of the proximal face (15) of the rib (13), the distal face (25) of which constitutes a subsequent splaying cam.

8. Device (5) according to one of Claims 1 to 7, **characterized in that** the first channel (10) has a proximal part of larger transverse dimension than the distal part, a side wall (12) of the proximal part of the first channel (10) forming a ramp over which the distal end (2c) of the anchoring bar (2) of a fastener can slide in order to cause the lateral displacement of said anchoring bar (2) towards the distal part of the first channel (10).

9. Device (5) according to one of Claims 1 to 8, **characterized in that** it has an approximately cylindrical shape, with a diameter less than 6 mm.

10. Device (5) according to Claim 9, **characterized in that** it includes a proximal cylindrical appendage (6) of smaller diameter, intended to be inserted into the distal end part of an instrument (1) for storing, dispensing and positioning surgical fasteners (A; A').

11. Device (5) according to one of Claims 1 to 10, **characterized in that** the distal end (9) of the device (5) includes a transverse wall (9a) substantially flanking the distal outlet of the first channel (10).

12. Instrument (1) for storing, dispensing and positioning recumbent I-shaped surgical fasteners, the fasteners having an anchoring bar (2), a stop bar (3) and a linking strip (4), the instrument (1) comprising a gripping means (B) provided with an actuator (29) that can move on a body (28) between a pushed-out position and a pushed-in position, an elongate tubular body (C) in which fasteners (A) are stored longitudinally one behind another, which tubular body (C) is fixed to the body (28) of the gripping means (B), **characterized in that** it further includes a device (5) for deploying and ejecting a fastener (A; A') according to one of Claims 1 to 11, which device is fixed to the distal end of the tubular body (C) so that the open half-space is located outside the tubular body (C).

13. Instrument (1) according to Claim 12, **characterized in that** it includes:
- a stationary component (62) housed in the tubular body (C) and a moving component (63) housed in the tubular body (C) so that it can be moved in longitudinal translation with respect to the stationary component (62) by means of an advancing rod (55) that can be actuated by the actuator (29), the stationary and moving components are positioned along a longitudinal face and each having notches (64) provided in said longitudinal face, with a pitch (P) corresponding to the length (L) of the stop bar (3) of a fastener (A; A'); and
- a slide (70) placed between the longitudinal faces of the stationary (62) and moving (63) components, having an approximately planar main part (71), a distal part (72) that bears against the proximal end (3b) of the stop bar (3) of the proximalmost fastener (A; A') and, in its proximal part, a first resilient tongue (73) projecting towards the moving component (63) and a second resilient tongue (74) projecting towards the stationary component (62), each capable of being engaged in a corresponding notch (64),
the geometry of the notches (64) being designed so that the displacement of the moving component (63) with respect to the stationary component (62) towards the distal end of the instrument (1) causes, by means of the first tongue (73) being engaged in a notch (64) of the moving component (63), the displacement of the slide (70) in the same direction, until the second tongue (74) of the slide (70) is engaged in a notch (64) of the stationary component (62), and so that the cooperation between the second tongue (74) and a notch (64) of the stationary component (62) prevents the slide (70) from returning towards the proximal end of the instrument (1) when the moving component (63) is displaced relative to the stationary component (62) towards the proximal end of the instrument (1).

14. Instrument (1) according to Claim 13, **characterized in that** the stationary and moving components (62, 63) have the form of semicylinders, the moving component (63) including a radial orifice (69) into which the curved end (56) of the advancing rod (55) is inserted.

15. Instrument (1) according to one of Claims 12 to 14, **characterized in that** it includes, fixedly housed in the distal part (C2) of the tubular body (C), a magazine (58) for storing the fasteners (A), which magazine includes a first longitudinal housing (59) that receives the aligned anchoring bars (2) of the fasteners and, contiguously, an ejection rod (54) that can be actuated by the actuator (29), a second longitudinal housing (60) that receives the aligned stop bars (3) of the fasteners, and a third housing (61), which joins the first and second housings (59, 60), for passage of the linking strips (4).

16. Instrument (1) according to one of claims 12 to 15, **characterized in that** the gripping means (B) includes:
- first and second levers (32, 35) that can be moved, by pushing in the actuator (29), between a proximal rest position and a distal end position, causing the longitudinal displacement in the distal direction of a rod (54) for ejecting the distalmost fastener out of the deployment/ejection device (5) and of a rod (55) for advancing the fasteners (A) in the tubular body (C), respectively;
- a member (43) mounted so as to pivot about a pin (44) attached to the body (28) of the gripping means (B), which member includes a tooth (45a) capable of cooperating with a rack (37) formed on one of the levers (32) during displacement of the actuator (29); and
- a flexible return means (47), one end of which is connected to the pivoting member (43) and the other end of which is connected to the body (28) of the gripping means (B),
the elastic return means (47) and the profile of the tooth (45a) and of the rack (37) being designed so that:
- when the actuator (29) is pushed in as far as an intermediate pushed-in position, the elastic return means (47) urges the pivoting member (43) towards a position such that the cooperation between the tooth (45a) of the pivoting member (43) and the rack (37) of the lever (32) prevents the actuator (29) from returning to the pushed-out position from said intermediate pushed-in position; and
- after the levers (32, 35) have reached their distalmost end position, upon releasing the actuator (29) as far as an intermediate release position, the flexible return means (47) urges the pivoting member (43) towards a position such that the cooperation between the tooth (45a) of the pivoting member (43) and the rack (37) of the lever (32) prevents the actuator (29) from being pushed in from said intermediate release position.

17. Instrument (1) according to Claim 16, **characterized in that** the rack (37) of the lever (32) includes, at its distal end, a tooth (38) of larger size and **in that** the pivoting member (43) includes, on one side, a finger (45) and, on the other side, a tab (46) that is capable of cooperating with a stop (75) attached to the body (28) of the gripping means (B) when the finger (45) comes into contact with the larger tooth (38), so that, when the actuator (28) has been pushed in, an elastic deformation of the tab (46) of the pivoting member (43) in contact with the stop (75) is necessary in order for the finger (45) to pass beyond said larger tooth (38).

## Patentansprüche

1. Vorrichtung (5), um eine chirurgische Klammer in Form eines liegenden "I" anzuwenden und auszugeben, wobei die Klammer einen Verankerungsstab (2), einen Anschlagsstab (3) und einen Verbindungsstab (4) aufweist, wobei die Vorrichtung (5) dazu vorgesehen ist, um angrenzend an das distale Ende eines Instrumentes (1) zum Speichern, Spenden und Anbringen von Klammern (A; A') positioniert zu werden, wobei die Vorrichtung eine Längsachse und ein distales Ende (9) aufweist, das sich in einer sich im Wesentlichen rechtwinklig zu der Längsachse verlaufenden Ebene befindet und einen ersten längs verlaufenden Kanal (10) beinhaltet, der an den proximalen und distalen Enden der Vorrichtung austritt, in dem der Verankerungsstab (2) einer Klammer in Längsrichtung bis zu einem Punkt gleiten kann, wo er an dem distalen Ende der Vorrichtung ausgegeben wird, wobei der erste Kanal (10) einen längs verlaufenden Schlitz (11) aufweist, der für das Hindurchtreten des Verbindungsstabes (4) der Klammer während des Gleitens vorgesehen ist,
wobei die Vorrichtung ferner Folgendes beinhaltet, nämlich
einen zweiten längs verlaufenden Kanal (20), in dem der Anschlagsstab (3) in Richtung des distalen Endes (9) der Vorrichtung (5) gleiten kann, wobei der zweite Kanal (20) im Wesentlichen in der gleichen Ebene wie der erste Kanal (10) und der Schlitz (11) des ersten Kanals liegt, wobei dieser an dem proximalen Ende der Vorrichtung austritt, und
ein erstes Steuerkurvenelement (19), das das distale Ende des zweiten Kanals (20) abschließt und ausgestaltet ist, um eine Anschlagsfläche für das distale Ende (3a) des Anschlagsstabes (3) zu bilden, und um ein Gleiten von dem distalen Ende (3a) in die entgegengesetzte Richtung zu dem ersten Kanal (10) zu ermöglichen, um eine Kippbewegung des Anschlagsstabes (3) zu bewirken,
**dadurch gekennzeichnet, dass** die Vorrichtung (5) eine im Wesentlichen längs verlaufende Auflagefläche (76) aufweist, die einen offenen Halbraum definiert, der nicht den ersten Kanal (10) enthält, und wobei sich der zweite Kanal (20) in den offenen Halbraum gegen die Auflagefläche (76) erstreckt;
und dass die Vorrichtung (5) ferner Folgendes beinhaltet, nämlich
- zumindest ein im Wesentlichen quer verlaufendes abgeschrägtes Steuerkurvenelement (15, 25), das proximal zu dem ersten Steuerkurvenelement (19) lokalisiert ist, welches in der Lage ist mit dem Verbindungsstab (4) und/oder dem Anschlagsstab (3) zusammenzuwirken, um eine laterale Schrägbewegung des proximalen Teils des Anschlagsstabes (3) weg von der Auflagefläche (76) zu bewirken; und
- ein Steuerkurvenelement (17) zur im Wesentlichen quer verlaufenden Entweichung, das zwischen dem ersten Steuerkurvenelement (19) und dem zumindest einen abgeschrägten Steuerkurvenelement (15, 25) lokalisiert ist, und in der Lage ist, mit dem distalen Teil des Anschlagsstabes (3) zusammenzuwirken, um eine laterale Verschiebung des distalen Teils des Anschlagsstabes (3) weg von der Auflagefläche (76) und dessen Loslösung von dem ersten Steuerkurvenelement ermöglicht.

2. Vorrichtung (5) gemäß Anspruch 1, **dadurch gekennzeichnet, dass** das erste Steuerkurvenelement (19) im Allgemeinen in einer Ebene liegt, die sich rechtwinklig zu der Auflagefläche (76) befindet und ausgehend von dem ersten Kanal (10) vom proximalen Ende der Vorrichtung (5) in Richtung des distalen Endes (9) geneigt ist.

3. Vorrichtung (5) gemäß Anspruch 1 oder 2, **dadurch gekennzeichnet, dass** das erste Steuerkurvenelement (19), betrachtet im Querschnitt in einer Längsebene rechtwinklig zu der Auflagefläche (76) eine konkave Form aufweist.

4. Vorrichtung (5) gemäß einem der Ansprüche 1 bis 3, **dadurch gekennzeichnet, dass** sie zwei abgeschrägte Steuerkurvenelemente (15, 25) aufweist, die ausgestaltet sind, um zwei aufeinanderfolgende laterale Schrägbewegungen des proximalen Teils des Anschlagsstabes (3) zu bewirken.

5. Vorrichtung (5) gemäß einem der Ansprüche 1 bis 4, **dadurch gekennzeichnet, dass** sie eine Längsrippe (13) beinhaltet, die im Wesentlichen in rechten Winkeln von der Auflagefläche (76) hervorsteht und die proximale Fläche (15) der Rippe eine erste Rampe bildet, die ein abgeschrägtes Steuerkurvenelement ausbildet.

6. Vorrichtung (5) gemäß Anspruch 5, **dadurch gekennzeichnet, dass** die Rippe (13) ein distales Teil (16) aufweist, das eine größere Höhe als deren proximales Teil (14) aufweist, wobei die proximale Fläche (17) des distalen Teils (16) der Rippe (13) eine zweite Rampe bildet, die ein distal zu der ersten Rampe (15) angeordnetes Steuerkurvenelement (17) zur Entweichung ausbildet.

7. Vorrichtung (5) gemäß Anspruch 5 oder 6, **dadurch gekennzeichnet, dass** es eine Aussparung (23) beinhaltet, die in der Auflagefläche (76) in einer Linie mit dem ersten Kanal (10) proximal der proximalen Fläche (15) der Rippe (13) ausgebildet ist,
wobei deren distale Fläche (25) ein folgendes abgeschrägtes Steuerkurvenelement ausbildet.

8. Vorrichtung (5) gemäß einem der Ansprüche 1 bis 7, **dadurch gekennzeichnet, dass** der erste Kanal (10) einen proximalen Teil mit größerem Quermaß als der distale Teil aufweist, wobei eine Seitenwand (12) des proximalen Teils des ersten Kanals (10) eine Rampe ausbildet, über die das distale Ende (2c) des Verankerungsstabes (2) einer Klammer gleiten kann, um die laterale Verschiebung des Verankerungsstabes (2) in Richtung des distalen Teils des ersten Kanals (10) zu bewirken.

9. Vorrichtung (5) gemäß einem der Ansprüche 1 bis 8, **dadurch gekennzeichnet, dass** sie eine im Wesentlichen zylindrische Form mit einem Durchmesser von kleiner als 6 mm aufweist.

10. Vorrichtung (5) gemäß Anspruch 9, **dadurch gekennzeichnet, dass** sie einen proximalen zylindrischen Fortsatz (6) mit einem kleineren Durchmesser beinhaltet, der dazu vorgesehen ist, um in das distale Endteil eines Instrumentes (1) zum Speichern, Spenden und Anbringen von chirurgischen Klammern (A; A') eingebracht zu werden.

11. Vorrichtung (5) gemäß einem der Ansprüche 1 bis 10, **dadurch gekennzeichnet, dass** das distale Ende (9) der Vorrichtung (5) eine Querwand (9a) beinhaltet, die im Wesentlichen den distalen Auslass des ersten Kanals (10) flankiert.

12. Instrument (1) zum Speichern, Spenden und Anbringen von chirurgischen Klammern von Form eines liegenden "I", wobei die Klammern einen Verankerungsstab (2), einen Anschlagsstab (3) und einen Verbindungsstab (4) aufweisen, wobei das Instrument (1) Folgendes aufweist, nämlich ein Greifelement (B), das mit einem Aktuator (29) versehen ist, der sich an einem Körper (28) zwischen einer ausgedrückten Position und einer eingedrückten Position bewegen kann, einen länglichen rohrförmigen Körper (C), in dem die Klammern (A) in Längsrichtung hintereinander gespeichert sind, wobei der rohrförmige Körper (C) an dem Körper (28) des Greifelementes (B) befestigt ist, **dadurch gekennzeichnet, dass** es ferner eine Vorrichtung (5) gemäß einem der Ansprüche 1 bis 11 beinhaltet, um eine Klammer (A; A') anzuwenden und auszugeben, wobei die Vorrichtung an dem distalen Ende des rohrförmigen Körpers (C) befestigt ist, so dass der offene Halbraum außerhalb des rohrförmigen Körpers (C) lokalisiert ist.

13. Instrument (1) gemäß Anspruch 12, **dadurch gekennzeichnet, dass** es Folgendes beinhaltet:
- eine stationäre Komponente (62), die in dem rohrförmigen Körper (C) untergebracht ist, und eine sich bewegende Komponente (63), die in dem rohrförmigen Körper (C) untergebracht ist, so dass sie in einer längs laufenden Translationsbewegung in Bezug auf die stationäre Komponente (62) mittels eines Vorschubstabes (55) bewegt werden kann, der durch den Aktuator (29) betätigt werden kann, wobei die stationären und sich bewegenden Komponenten entlang einer Längsseite angeordnet sind und jeweils Ausnehmungen (64) aufweisen, die in der Längsseite vorgesehen sind, und einen Abstand (P) aufweisen, der der Länge (L) des Anschlagsstabes (3) einer Klammer (A; A') entspricht; und
- ein Gleitstück (70), das zwischen den Längsseiten der stationären (62) und der sich bewegenden (63) Komponenten angeordnet ist, das einen im Wesentlichen planen Hauptteil (71), einen distalen Teil (72), der auf dem proximalen Ende (3b) des Anschlagsstabes (3) der am meisten proximal liegenden Klammer (A; A') anliegt, und in dessen proximalen Teil eine erste Federzunge (73), die in Richtung der sich bewegenden Komponente (63) hervorsteht, und eine zweite Federzunge (74), die in Richtung der stationären Komponente (62) hervorsteht, aufweist, wobei jede in der Lage ist mit einer entsprechenden Ausnehmung (64) in Eingriff zu sein,
wobei die Geometrie der Ausnehmungen (64) derart ausgestaltet ist, dass die Verschiebung der sich bewegenden Komponente (63) in Bezug auf die stationäre Komponente (62) in Richtung des distalen Endes des Instrumentes (1) mittels der ersten Zunge (73), die sich in Eingriff mit einer Ausnehmung (64) der sich bewegenden Komponente (63) befindet, die Verschiebung des Gleitstückes (70) in die gleiche Richtung bewirkt, bis die zweite Zunge (74) des Gleitstückes (70) in Eingriff mit einer Ausnehmung (64) der stationären Komponente (62) steht, und so dass das Zusammenwirken zwischen der zweiten Zunge (74) und einer Ausnehmung (64) der stationären Komponente (62) verhindert, dass das Gleitstück (70) in Richtung des proximalen Endes des Instrumentes (1) zurückkehrt, wenn die sich bewegende Komponente (63) relativ zu der stationären Komponente (62) in Richtung des proximalen Endes des Instrumentes (1) verschoben wird.

14. Instrument (1) gemäß Anspruch 13, **dadurch gekennzeichnet, dass** die stationäre und sich bewegende Komponenten (62, 63) die Form von Halbzylindern aufweisen, wobei die sich bewegende Komponente (63) eine radiale Öffnung (69) beinhaltet, in die das gebogene Ende (56) des Vorschubstabes (55) eingebracht wird.

15. Instrument (1) gemäß einem der Ansprüche 12 bis 14, **dadurch gekennzeichnet, dass** es Folgendes beinhaltet, nämlich ein in dem distalen Teil (C2) des rohrförmigen Körpers (C) auf befestigte Art und Weise untergebrachtes Magazin (58) zum Speichern der Klammern (A), wobei das Magazin Folgendes beinhaltet, nämlich ein erstes längs verlaufendes Gehäuse (59), das die ausgerichteten Verankerungsstäbe (2) der Klammern aufnimmt, und angrenzend einen Ausgabestab (54), der durch den Aktuator (29) betätigt werden kann, ein zweites längs verlaufendes Gehäuse (60), das die ausgerichteten Anschlagsstäbe (3) der Klammern aufnimmt, und ein drittes Gehäuse (61), das sich an das erste und zweite Gehäuse (59, 60) anschließt, um die Verbindungsstäbe (4) hindurchtreten zu lassen.

16. Instrument (1) gemäß einem der Ansprüche 12 bis 15, **dadurch gekennzeichnet, dass** das Greifelement (B) Folgendes beinhaltet:
- erste und zweite Hebel (32, 35), die durch Eindrücken des Aktuators (29) zwischen einer proximalen Ruheposition und einer distalen Endposition bewegt werden können, wodurch die Längsverschiebung in die distale Richtung eines Stabes (54) zur Ausgabe der am meisten distal gelegenen Klammer aus der Vorrichtung zur Anwendung und Ausgabe (5), bzw. eines Stabes (55) zum Vorschub der Klammern (A) in den rohrförmigen Körper (C) bewirkt wird;
- ein Teil (43), das derart angebracht ist, so dass es sich um einen Stift (44) drehen kann, der an dem Körper (28) des Greifelementes (B) befestigt ist, wobei das Teil einen Zahn (45a) beinhaltet, der in der Lage ist mit einer Zahnstange (37), die auf einem der Hebel (32) ausgebildet ist, während der Verschiebung des Aktuators (29) zusammenzuwirken; und
- ein flexibles Rückführmittel (47), wovon ein Ende mit dem Drehteil (43) verbunden ist, und dessen anderes Ende mit dem Körper (28) des Greifelementes (B) verbunden ist, wobei das elastische Rückführmittel (47) und das Profil des Zahnes (45a) und der Zahnstange (37) derart ausgestaltet sind, so dass Folgendes gilt:
- wenn der Aktuator (29) bis zu einer eingedrückten Zwischenposition eingedrückt ist, zwingt das elastische Rückführmittel (47) das Drehteil (43) derart in eine Richtung, dass das Zusammenwirken zwischen dem Zahn (45a) des Drehteils (43) und der Zahnstange (37) des Hebels (32) verhindert, dass der Aktuator (29) in die ausgedrückte Position von der eingedrückten Zwischenposition zurückkehrt; und
- nachdem die Hebel (32, 35) ihre am meisten distal liegende Endpositionen erreicht haben, zwingt das flexible Rückführmittel (47), nachdem der Aktuator (29) bis zu einer Entlastungszwischenposition entlastet wird, das Drehteil (43) in Richtung einer Position, so dass das Zusammenwirken zwischen dem Zahn (45a) des Drehteiles (43) und der Zahnstange (37) des Hebels (32) verhindert, dass der Aktuator (29) aus seiner entlasteten Zwischenposition eingedrückt wird.

17. Instrument (1) gemäß Anspruch 16, **dadurch gekennzeichnet, dass** die Zahnstange (37) des Hebels (32) an deren distalen Ende einen größeren Zahn (38) beinhaltet, und dass das Drehteil (43) an einer Seite einen Finger (45) und auf der anderen Seite eine Nase (46) beinhaltet, die in der Lage ist mit einem Anschlag (75) zusammenzuwirken, der an dem Körper (28) des Greifelementes (B) befestigt ist, wenn der Finger (45) mit dem größeren Zahn (38) in Kontakt kommt, so dass, wenn der Aktuator (28) eingedrückt wurde, eine elastische Deformierung der Nase (46) des Drehteiles (43), das mit dem Anschlag (75) in Kontakt steht, erforderlich ist, damit der Finger (45) jenseits des größeren Zahnes (38) treten kann.

## Revendications

1. Dispositif (5) de déploiement et d'éjection d'une attache chirurgicale en I couché, comportant une barre d'ancrage (2), une barre d'arrêt (3) et une barrette de liaison (4), le dispositif (5) étant destiné à être fixé à l'extrémité distale d'un appareil (1) de stockage, de distribution et de pose de telles attaches (A ; A'), le dispositif présentant un axe longitudinal et une extrémité distale (9) disposée selon un plan sensiblement orthogonal à l'axe longitudinal, et comportant un premier canal (10) longitudinal débouchant aux extrémités proximale et distale du dispositif, dans laquelle la barre d'ancrage (2) d'une attache peut coulisser longitudinalement jusqu'à son éjection à l'extrémité distale du dispositif, le premier canal (10) présentant une fente longitudinale (11) destinée au passage de la barrette de liaison (4) de ladite attache lors dudit coulissement, le dispositif comprenant en outre :
- un deuxième canal (20) longitudinal dans lequel la barre d'arrêt (3) peut coulisser vers l'extrémité distale (9) du dispositif (5), le deuxième canal (20) étant disposé sensiblement dans le même plan que le premier canal (10) et la fente (11) du premier canal, débouchant à l'extrémité proximale du dispositif,
- une première came (19) obturant l'extrémité distale du deuxième canal (20), agencée pour former une surface de butée pour l'extrémité distale (3a) de la barre d'arrêt (3) et permettre le glissement de ladite extrémité distale (3a) en direction opposée au premier canal (10), de sorte à produire un pivotement de la barre d'arrêt (3),
**caractérisé en ce que** le dispositif (5) présente une face d'appui (76) sensiblement longitudinale définissant un demi espace ouvert ne contenant pas le premier canal (10), et dans lequel le deuxième canal (20) se prolonge dans le demi espace ouvert contre la face d'appui (76),
et **en ce que** le dispositif (5) comprend en outre :
- au moins une came d'écartement (15, 25), sensiblement transversale, située en amont de la première came (19), apte à coopérer avec la barrette de liaison (4) et/ou la barre d'arrêt (3) pour provoquer un écartement latéral de la partie proximale de la barre d'arrêt (3) à l'opposé de la face d'appui (76) ;
- une came d'échappement (17), sensiblement transversale, située entre la première came (19) et la ou les cames d'écartement (15, 25), apte à coopérer avec la partie distale de la barre d'arrêt (3) pour permettre le déplacement latéral de la partie distale de la barre d'arrêt (3) à l'opposé de la face d'appui (76), et son dégagement hors de la première came.

2. Dispositif (5) selon la revendication 1, **caractérisé en ce que** la première came (19) est globalement située dans un plan orthogonal à la face d'appui (76) et est inclinée, lorsque l'on s'éloigne du premier canal (10), de l'extrémité proximale du dispositif (5) en direction de l'extrémité distale (9).

3. Dispositif (5) selon la revendication 1 ou 2, **caractérisé en ce que** la première came (19) présente, vue en section dans un plan longitudinal orthogonal à la face d'appui (76), une forme concave.

4. Dispositif (5) selon l'une des revendications 1 à 3, **caractérisé en ce qu'**il comprend deux cames d'écartement (15, 25) agencées pour provoquer deux écartements latéraux séquencés de la partie proximale de la barre d'arrêt (3).

5. Dispositif (5) selon l'une des revendications 1 à 4, **caractérisé en ce qu'**il comprend une nervure longitudinale (13) faisant saillie sensiblement perpendiculairement de la face d'appui (76), et dont la face proximale (15) forme une première rampe constituant une came d'écartement.

6. Dispositif (5) selon la revendication 5, **caractérisé en ce que** la nervure (13) présente une partie distale (16) de plus grande hauteur que sa partie proximale (14), la face proximale (17) de la partie distale (16) de la nervure (13) formant une deuxième rampe qui, située en aval de la première rampe (15), constitue la came d'échappement (17).

7. Dispositif (5) selon la revendication 5 ou 6, **caractérisé en ce qu'**il comprend une cavité (23) formée dans la face d'appui (76), au droit du premier canal (10), en amont de la face proximale (15) de la nervure (13), dont la face distale (25) constitue une came d'écartement subséquente.

8. Dispositif (5) selon l'une des revendications 1 à 7, **caractérisé en ce que** le premier canal (10) présente une partie proximale de plus grande dimension transversale que sa partie distale, une paroi latérale (12) de la partie proximale du premier canal (10) formant une rampe sur laquelle peut glisser l'extrémité distale (2c) de la barre d'ancrage (2) d'une attache pour provoquer le déplacement latéral de ladite barre d'ancrage (2) vers la partie distale du premier canal (10).

9. Dispositif (5) selon l'une des revendications 1 à 8, **caractérisé en ce qu'**il présente une forme sensiblement cylindrique, de diamètre inférieur à 6 mm.

10. Dispositif (5) selon la revendication 9, **caractérisé en ce qu'**il comporte un appendice cylindrique proximal (6) de plus faible diamètre, destiné à être introduit dans la partie extrême distale d'un appareil (1) de stockage, de distribution et de pose d'attaches chirurgicales (A ; A').

11. Dispositif (5) selon l'une des revendications 1 à 10, **caractérisé en ce que** l'extrémité distale (9) du dispositif (5) comporte une paroi transversale (9a) s'élargissant substantiellement autour du débouché distal du premier canal (10).

12. Appareil (1) de stockage, de distribution et de pose d'attaches chirurgicales en I couché, comportant une barre d'ancrage (2), une barre d'arrêt (3) et une barrette de liaison (4), ledit appareil (1) comportant un moyen de préhension (B) muni d'une poignée (29) d'actionnement mobile sur un corps (28) entre une position sortie et une position enfoncée, un corps tubulaire (C) allongé dans lequel sont stockées longitudinalement des attaches (A) les unes derrière les autres, fixé au corps (28) du moyen de préhension (B) , **caractérisé en ce qu'**il comporte en outre un dispositif (5) de déploiement et d'éjection d'une attache (A ; A') selon l'une des revendications 1 à 11, fixé à l'extrémité distale du corps tubulaire (C) de sorte que le demi espace ouvert soit situé à l'extérieur du corps tubulaire (C).

13. Appareil (1) selon la revendication 12, **caractérisé en ce qu'**il comporte :
- une pièce (62) logée dans le corps tubulaire (C) de façon fixe et une pièce mobile (63) logée dans le corps tubulaire (C) de façon à pouvoir être déplacée en translation longitudinale par rapport à la pièce fixe (62) au moyen d'une tige d'avancée (55) actionnable par la poignée (29), les pièces fixe et mobile étant disposées de manière adjacente le long d'une face longitudinale et présentant chacune des crans (64) ménagés dans ladite face longitudinale, selon un pas (P) correspondant à la longueur (L) de la barre d'arrêt (3) d'une attache (A ; A') ;
- un coulisseau (70) disposé entre les faces longitudinales des pièces fixe (62) et mobile (63), comportant une partie principale (71) sensiblement plane, une partie distale (72) en appui contre l'extrémité proximale (3b) de la barre d'arrêt (3) de l'attache (A ; A') la plus en amont et, à sa partie proximale, une première languette (73) élastique en saillie vers la pièce mobile (63) et une deuxième languette (74) élastique en saillie vers la pièce fixe (62), aptes à être engagées chacune dans un cran (64) correspondant,
la géométrie des crans (64) étant prévue pour que le déplacement de la pièce mobile (63) par rapport à la pièce fixe (62) vers l'extrémité distale de l'appareil (1) entraîne, grâce à la première languette (73) engagée dans un cran (64) de la pièce mobile (63), le déplacement du coulisseau (70) dans le même sens, jusqu'à ce que la deuxième languette (74) du coulisseau (70) soit engagée dans un cran (64) de la pièce fixe (62), et pour que la coopération entre la deuxième languette (74) et un cran (64) de la pièce fixe (62) empêche le retour du coulisseau (70) vers l'extrémité proximale de l'appareil (1) lorsque la pièce mobile (63) est déplacée par rapport à la pièce fixe (62) vers l'extrémité proximale de l'appareil (1).

14. Appareil (1) selon la revendication 13, **caractérisé en ce que** les pièces fixe et mobile (62, 63) présentent la forme de demi cylindres, la pièce mobile (63) comportant un orifice radial (69) dans lequel est insérée l'extrémité recourbée (56) de la tige d'avancée (55).

15. Appareil (1) selon l'une des revendications 12 à 14, **caractérisé en ce qu'**il comporte, logé dans la partie distale (C2) du corps tubulaire (C) de façon fixe, un magasin (58) de stockage des attaches (A) qui comporte un premier logement (59) longitudinal recevant les barres d'ancrage (2) alignées des attaches et, de façon contiguë, une tige d'éjection (54) actionnable par la poignée (29), un deuxième logement (60) longitudinal recevant les barres d'arrêt (3) alignées des attaches, et un troisième logement (61) reliant les premier et deuxième logements (59, 60) pour le passage des barrettes de liaison (4).

16. Appareil (1) selon l'une des revendications 12 à 15, **caractérisé en ce que** le moyen de préhension (B) comprend :
- un premier et un deuxième leviers (32, 35) aptes à être déplacés, par l'enfoncement de la poignée (29) d'actionnement, entre une position de repos amont et une position extrême aval, en provoquant le déplacement longitudinal dans le sens distal respectivement d'une tige d'éjection (54) de l'attache la plus en aval hors du dispositif (5) de déploiement et d'éjection et d'une tige d'avancée (55) des attaches (A) dans le corps tubulaire (C) ;
- un organe (43) monté pivotant autour d'un axe (44) solidaire du corps (28) du moyen de préhension (B), comportant une dent (45a) apte à coopérer avec une denture (37) ménagée sur l'un des leviers (32), lors du déplacement de la poignée (29) ;
- un moyen de rappel élastique (47) dont une extrémité est reliée à l'organe pivotant (43) et dont l'autre extrémité est reliée au corps (28) du moyen de préhension (B) ;
le moyen de rappel élastique (47) et le profil de la dent (45a) et de la denture (37) étant agencés pour que :
- lors de l'enfoncement de la poignée (29) jusqu'à une position d'enfoncement intermédiaire, le moyen de rappel élastique (47) sollicite l'organe pivotant (43) vers une position telle que la coopération entre la dent (45a) de l'organe pivotant (43) et la denture (37) du levier (32) empêche le retour de la poignée (29) en position sortie depuis ladite position d'enfoncement intermédiaire ;
- après que les leviers (32, 35) ont atteint leur position extrême aval, lors du relâchement de la poignée (29) jusqu'à une position de relâchement intermédiaire, le moyen de rappel élastique (47) sollicite l'organe pivotant (43) vers une position telle que la coopération entre la dent (45a) de l'organe pivotant (43) et la denture (37) du levier (32) empêche l'enfoncement de la poignée (29) depuis ladite position de relâchement intermédiaire.

17. Appareil (1) selon la revendication 16, **caractérisé en ce que** la denture (37) du levier (32) comprend, à son extrémité distale, une dent (38) de taille supérieure, et **en ce que** l'organe pivotant (43) comprend d'une part un doigt (45) et d'autre part une patte (46) apte à coopérer avec une butée (75) solidaire du corps (28) du moyen de préhension (B) lorsque le doigt (45) vient en contact avec la dent (38) de taille supérieure, de sorte que le passage du doigt (45) au-delà de ladite dent (38) de taille supérieure suite à l'enfoncement de la poignée (29) nécessite une déformation élastique de la patte (46) de l'organe pivotant (43) au contact de la butée (75).
